# EUROPEAN PATENT SPECIFICATION

(11) **EP 2 356 968 B1**
(45) Date of publication and mention of the grant of the patent: **20.06.2012**
(21) Application number: 10157549.6
(22) Date of filing: 24.03.2010
(51) Int. Cl.: A61K 8/02, A61K 8/19, A61K 8/25, A61K 8/46, A61K 8/81, A61Q 9/04

(54) **Substrate-based depilatory article**
Article d'épilation à base de substrat
Enthaarungsartikel auf Substratbasis

(30) Priority: 17.02.2010 US 305178 P
(43) Date of publication of application: 17.08.2011
(73) Proprietor: The Procter & Gamble Company, Cincinnati, OH 45202 (US)
(72) Inventor: Smith, Paul James, Whitton, Middlesex TW2 6EB (GB); Watts, Graeme, William, Woking, Surrey GU24 0AW (GB); Fletcher, Jamie, Anthony, Luston, Hertfordshire HR6 OEB (GB); Dring, Neil, Charles, Medmenham, Buckinghamshire SL7 2EZ (GB); Sagel, Paul, Albert, Maineville, OH 45039 (US); Passi, Rajeev, Kumar, West Chester, OH 45069 (US); Mitra, Shekhar, Cincinnati, OH 45243 (US)
(74) Representative: Simpson, Kirsty Mairi

(56) References cited:
- EP-A1- 1 736 207
- WO-A1-2004/014179
- WO-A1-2006/136440
- CA-A1- 2 354 829
- DE-A1- 2 253 117
- GB-A- 1 291 377
- GB-A- 2 367 749
- JP-A- 6 135 825
- JP-A- 9 103 320
- JP-A- 2004 305 310
- US-A1- 2004 219 118
- DATABASE WPI Week 200542 Thomson Scientific, London, GB; AN 2005-410646 XP002592239 & JP 2005 145895 A (KAO CORP) 9 June 2005 (2005-06-09)
- DATABASE GNPD [Online] MINTEL 01 September 2009 'Sensitive Skin Hair Removal Wax Strips' Database accession no. 1168092
- DATABASE GNPD [Online] MINTEL 01 June 2006 'In-Shower Hair Removal Cream' Database accession no. 546424
- DATABASE GNPD [Online] MINTEL 01 June 2009 'Extra Strength Hair Remover Lotion' Database accession no. 1117973
- DATABASE GNPD [Online] MINTEL 01 February 2008 'Cream Hair Remover' Database accession no. 863120

## Description

### FIELD OF THE INVENTION

The present invention relates to depilatory articles comprising a chemically active depilatory composition disposed on a substrate.

### BACKGROUND OF THE INVENTION

Depilatory compositions used to remove unwanted hair by chemical activity are known. Such compositions may comprise reducing agents to degrade keratin in the hair and thus weaken the hair strands. These compositions may take the form of creams, lotions and the like which may be applied to the unwanted hair in a variety of ways, such as with a spatula. The spatula or another suitable implement is then used to scrape off the weakened hair strands and complete the depilation process. This can be a messy and awkward procedure for the user of the depilatory cream or lotion. By disposing the depilatory composition on a substrate one may overcome or mitigate such disadvantages. Substrate-based depilatory products are known from JP63073910A, US2006002878, JP6135826A, JP11012123A JP62230711A, and EP1736207.

While addressing the handling problems of creams and lotions, substrate-based depilatory compositions typically provide a lower dosage of active ingredients to the unwanted hair than creams or lotions, thus reducing their efficacy versus a lotion or cream. In investigating this matter, applicants have established that thioglycolate salts comprising monovalent metal cations, such as potassium thioglycolate, are effective at removing hair from the skin, even at low doses, but may expose the skin tissue to harsh chemical conditions, resulting in irritation. On the other hand, thioglycolate salts comprising divalent metal cations, such as calcium thioglycolate, are relatively non-irritating to the skin, but at the low doses achievable in substrate-based products, they have, in the past, not been sufficiently effective to provide adequate depilation. Without wishing to be bound by theory, the applicants believe that the presence of monovalent metal cations causes the pH of the composition to increase *in situ* because the system is not buffered, with higher pH leading to skin irritation. In addition, the applicants believe that monovalent thioglycolate salts penetrate the skin faster than divalent metal salts and thereby may cause greater irritation. Irritancy issues may be exacerbated if water is lost from the composition before or during use as this increases the concentration of potentially irritating ingredients. Accordingly, there exists a need for a substrate-based depilatory product that provides both a desirable level of depilatory action and is mild enough to avoid irritation of the skin.

### SUMMARY OF THE INVENTION

According to a first aspect of the invention, the applicants have surprisingly found that a depilatory article according to claim 1 meets the aforementioned need by providing a desirable level of depilatory efficacy in a substrate-based application while simultaneously being mild enough to avoid skin irritation.

According to a second aspect of the invention, a cosmetic method of removing hair from the skin is provided, comprising the steps of: applying a depilatory article according to the first aspect of the invention to a surface of mammalian skin, preferably human skin, leaving said depilatory article in contact with the skin for a period of at least 1 minute, preferably 2 to 10 minutes, more preferably 2 to 8 minutes, removing said depilatory article from the surface of the skin, and preferably rubbing, scraping, rinsing or wiping the surface of the skin in the area to which the depilatory article was applied.

According to a third aspect of the invention, a depilatory kit is also provided, comprising: a depilatory article according to the first aspect of the invention, optionally at least one of a pre-treatment skin care composition, a post-treatment skin care composition and/or a tool to assist removal of hair and/or aqueous depilatory composition after use, and packaging for said depilatory kit.

### BRIEF DESCRIPTION OF THE DRAWINGS

Fig.1. is a plan view of a depilatory article of the present invention.
Fig.2. is a side view of a depilatory article of the present invention.
Fig.3. is a side view of a depilatory article of the present invention applied to keratinous tissue.

### DETAILED DESCRIPTION OF THE INVENTION

As used herein, the term "buffering base" refers to a base capable of opposing pH changes by means of chemical or physical (solubility) processes and thereby limiting the pH to less than or equal to 13.

As used herein, the term "water impermeable" includes materials or objects through which water in its liquid state does not pass.

As used herein, the term "sodium silicate" refers to Na₂SiO₃, any other silicate comprising sodium as the only cation besides silicon, and any other silicate comprising sodium. The same definition applies correspondingly to any other silicate, for example "potassium silicate" refers to K₂SiO₃, any other silicate comprising potassium as the only cation besides silicon and any other silicate comprising potassium, "ammonium silicate" to (NH₄)₂SiO₃, any other silicate comprising ammonium as the only cation besides silicon and any other silicate comprising ammonium and "manganese silicate" to Mn₂SiO₄, any other silicate comprising manganese as the only cation besides silicon and any other silicate comprising manganese.

Depilatory articles of the present invention comprise a water impermeable substrate, to facilitate application of the aqueous depilatory composition to keratinous tissue and prevent a messy usage experience. Using a water impermeable substrate also prevents water loss from the aqueous depilatory composition while the aqueous depilatory composition is in contact with the keratinous tissue and thus prevents the aqueous depilatory composition from drying out. Water loss from the aqueous depilatory composition lowers the water concentration, thus increasing the concentration of active ingredients and bases present. This could result in irritation to the skin, which applicants wish to avoid.

Depilatory articles of the present invention comprise an aqueous depilatory composition in contact with a surface of the water impermeable substrate, forming a coated region of the water impermeable substrate. The aqueous depilatory composition should be suitable for being placed in contact with a user's skin (and unwanted hair). As a result of the lowered efficacy upon making an acceptable substrate-based depilatory product, a further change must be made to the composition in order to provide a desirable level of depilatory activity. Accordingly, The aqueous depilatory composition comprises at least one water-soluble silicate.

Preferably, the aqueous depilatory composition comprises at least one water-soluble silicate selected from lithium silicates; sodium silicates (including disodium metasilicate pentahydrate and disodium metasilicate nanohydrate); potassium silicates; ammonium silicates; imidazolium silicates, synthetic silicates (clays) or mixtures thereof. More preferably, the aqueous depilatory composition comprises at least one water-soluble silicate selected from synthetic clays; sodium silicates, potassium silicates, or mixtures thereof and even more preferably the aqueous depilatory composition comprises a sodium silicate or mixtures of sodium silicates.

Suitable synthetic and natural silicates (clays) are available commercially as: Laponite® RDS; XLS and S etc. (available from RockWood Additives Limited).

Without wishing to be bound by theory, the applicants believe that the water-soluble silicate possesses an anionic surface charge that enhances the dissociation of the thioglycolate salt via an attractive force imposed upon the cation in said thioglycolate salt. This increases the amount of conjugate acid formed from the thioglycolate salt and therefore increases the effectiveness of the aqueous depilatory composition. Additionally, any monovalent cations such as those present in the water-soluble silicate or derived from monovalent cation containing salts are able to displace the cation of the thioglycolate salt and further enhance dissociation of said thioglycolate salt without leading to the large pH increases associated with poor safety characteristics among compositions of the prior art. Accordingly, the presence of some monovalent metal cations in the aqueous depilatory compositions is essential.

*In situ,* the silicate comprised within the aqueous depilatory composition forms silicate salt, which hydrates and tends to remove water from the composition. Since the silicate salt is colloidal, the colloidal solids may provide surfaces for evaporation and this evaporation, in turn, may be enhanced at the surface of the coated region intended to be placed in contact with the skin, which otherwise is a surface that may be open to the atmosphere. As indicated above, evaporation or other loss of water from the aqueous depilatory composition leads to a pH change that may lead to skin irritation which it is desirable to avoid. Applicants believe that, within certain dosage-per-unit-area ranges, the effects referred to above may be kept within acceptable limits. As a result, the silicate is preferably present in the aqueous depilatory composition in an amount per unit area of the coated region of from 2.05 x 10⁻⁸ mol/cm² to 1.23 x 10⁻⁴ mol/cm², preferably from 1.64 x 10⁻⁷ mol/cm² to 3.69 x 10⁻⁵ mol/cm² and more preferably from 4.92 x 10⁻⁷ mol/cm² to 8.20 x 10⁻⁶ mol/cm².

The aqueous depilatory composition comprises at least one thioglycolate salt acting as a hair removal agent when the aqueous depilatory composition is applied to unwanted hair. Preferably, the aqueous depilatory composition comprises sodium, potassium, magnesium, calcium, beryllium, strontium, zinc, monoethanolamine, ammonium, tetralkylammonium, imidazolium, pyridinium, phosphonium or glyceryl thioglycolate salts, or mixtures thereof, which may include dianion forms of thioglycolate. More preferably, the aqueous depilatory composition comprises at least one of sodium, potassium, magnesium or calcium thioglycolate, or mixtures thereof. Even more preferably the aqueous depilatory composition comprises potassium or calcium thioglycolate, or mixtures thereof. In a preferred embodiment, the concentration of the conjugate acid of the thioglycolate salt is from 0.5% to 12.0%, more preferably from 0.8% to 8.0% and even more preferably from 1.0% to 6.0% by weight of the aqueous depilatory composition.

The aqueous depilatory composition comprises a divalent metal cation, preferably wherein the thioglycolate salt, the buffering base (if present) or both comprises a divalent metal cation. In another preferred embodiment, the thioglycolate salt comprises a divalent metal cation. Applicants have established that thioglycolate salts comprising monovalent metal cations, such as potassium thioglycolate, are effective at removing hair from the skin, even at low doses, but may expose the skin tissue to harsh chemical conditions, resulting in irritation. On the other hand, thioglycolate salts comprising divalent metal cations, such as calcium thioglycolate, are relatively non-irritating to the skin.

The aqueous depilatory composition comprises a monovalent cation, preferably a monovalent metal cation. Without wishing to be bound by theory, the applicants believe that the presence of monovalent metal cations increases the dissociation of thioglycolate salts. The monovalent cations such as those derived from monovalent cation containing salts are able to displace the cation of the thioglycolate salt and further enhance dissociation of said thioglycolate salt. This increases the amount of deprotonated thioglycolate formed from the thioglycolate salt and therefore increases the effectiveness of the aqueous depilatory composition. Sources of monovalent cations include potassium, sodium, lithium, ammonium, tetraalkyl ammonium and imidazolium salts, which may be a component of another ingredient, for example a thickening system or skin care active. Preferred sources of monovalent cations include potassium and sodium salts.

In order to further enhance the safety of the resulting product, it is advantageous to limit the amount of monovalent cations, preferably monovalent metal cations, to which the skin is exposed when the depilatory article is used, although a small quantity may improve the efficacy of the aqueous depilatory composition. Advantageously, the quantity of monovalent cations (or monovalent metal cations in the preferred embodiment above) per unit area of the aforementioned coated region is less than 5.10 x 10⁻⁴ mol/cm², preferably less than 3 x 10⁻⁴ mol/cm⁻², more preferably from 1 x 10⁻⁹ mol/cm² to 1.5 x 10⁻⁴ mol/cm², even more preferably from 2.50 x 10⁻⁸ mol/cm² to 6.65 x 10⁻⁵ mol/cm² and even more preferably still from 6 x 10⁻⁷ mol/cm² to 4.5 x 10⁻⁵ mol/cm². The selection of thiglycolate salt and optional ingredients including the base may be made considering the quantity of monovalent cations or monovalent metal cations achieved.

In an aqueous depilatory composition comprising a mixture of monovalent and divalent ions, controlling the ratio of divalent ions to monovalent ions may also improve the safety characteristics of the depilatory articles of the present invention. Increasing the concentration of divalent ions relative to the concentration of monovalent ions increases the likelihood that any particular depilatory active species is associated with a divalent ion, rather than the more irritating monovalent ions. On the other hand, increasing the concentration of monovalent ions increases the effectiveness of the aqueous depilatory composition. Accordingly, in an alternative embodiment the ratio of the concentration of divalent ions to the concentration of monovalent ions present in the aqueous depilatory composition is advantageously in the range of from 400:1 to 0.02:1, preferably from 200:1 to 0.1:1, more preferably 60:1 to 0.3:1, even more preferably from 20:1 to 0.5:1, and even more preferably still from 15:1 to 1:1.

The pH of the aqueous depilatory composition may advantageously be in the range of from 6 to 13.8, preferably from greater than 7 to 13, more preferably from 9 to 12.9, even more preferably from 10 to 12.8, even more preferably still from 12 to 12.7 and yet more preferably from 12.3 to 12.6 to improve the efficacy of the active ingredient. The aqueous depilatory composition may, in a preferred embodiment, comprise at least one base to control the pH. Preferably, the aqueous depilatory composition comprises potassium hydroxide; sodium hydroxide; lithium hydroxide; calcium hydroxide; barium hydroxide; caesium hydroxide; sodium hydroxide; ammonium hydroxide; strontium hydroxide; rubidium hydroxide; magnesium hydroxide; zinc hydroxide; sodium carbonate; pyridine; ammonia; alkanolamides (including monoethanolamine, diethanolamine, triethanolamine), phosphates (including tetrasodium phosphate), arginine or mixtures thereof. More preferably, the aqueous depilatory composition comprises at least one buffering base, even more preferably the aqueous depilatory composition comprises calcium hydroxide, magnesium hydroxide; barium hydroxide; strontium hydroxide; zinc hydroxide; arginine or mixtures thereof. Still more preferably the aqueous depilatory composition comprises calcium hydroxide; magnesium hydroxide, zinc hydroxide, sodium hydroxide, potassium hydroxide or mixtures thereof. Even more preferably still, the aqueous depilatory composition comprises calcium hydroxide.

In a preferred embodiment, the base is present at a concentration of from 0.1 % to 10.0%, more preferably from 0.5% to 8.0% and even more preferably from 1.0% to 5.0%, by weight of the aqueous depilatory composition.

The water impermeable substrate preferably comprises at least one water impermeable material and is compatible with depilatory compositions. Examples of useful water impermeable materials include but are not limited to polypropylene (PP); polyethylene (PE, including HDPE and LLDPE); polyethylene terephthalate (PET); polyvinylchloride (PVC); polyamide (PA); polycarbonate; polyurethane; cellulose acetate; polychloropene; polysulfone; polytetrafluoroethylene (PTFE); polyvinyl acetate (PVA); polystyrene; polyphenylene oxide (PPO); acrylonitrile butadiene styrene (ABS); acrylic; acrylonitrile styrene acrylate (ASA); ethylene vinyl alcohol (EVA); natural rubber, latex, nylon, nitrile, silicone and thermo plastic elastomers (TPE). The substrate may comprise a single polymer or mixtures of polymers or copolymers. Preferably the substrate comprises a plastic sheet, more preferably a polyolefin, even more preferably a polyethylene and even more preferably still high density polyethylene.

In an advantageous embodiment, the aqueous depilatory composition is disposed upon the water impermeable material, preferably plastic sheet, more preferably polyolefin, even more preferably polyethylene and even more preferably still high density polyethylene. In this advantageous embodiment, there is preferably no layer of water permeable material between the aqueous depilatory composition and the water impermeable material. In a preferred embodiment, the water impermeable material forms a water impermeable layer.

The water impermeable substrate preferably possesses a rigidity in the range of from 5.00 g/cm to 0.08 g/cm, more preferably from 3.00 g/cm to 0.08 g/cm, even more preferably from 1.80 g/cm to 0.10 g/cm, even more preferably still from 0.80 g/cm to 0.15 g/cm and yet more preferably from 0.60 g/cm to 0.25 g/cm. This rigidity of the water impermeable substrate ensures that desirable handleability and conformability attributes of a depilatory article are achieved. In particular, the article collapsing under gravity or folding is avoided, which is especially undesirable if different areas of the aqueous depilatory composition are able to readily come into contact with each other, while maintaining the capability for the substrate to conform to the surface to which it is applied without folding or crinkling, in order to further improve depilatory efficiency. Accordingly, the substrate is readily conformable to the skin and unwanted hair without permanently deforming during use, as this may also result in problems for the user during application. In a preferred embodiment, the rigidity is substantially constant and does not change during the lifetime of a product.

Rigidity can be readily measured using the American Standard Test Method (ASTM) D2923-06 on a Handle-O-Meter, model #211-300, available from Thwing-Albert Instrument Co. of Philadelphia, Pa. The rigidity is read directly from the meter and expressed as grams per centimetre of sample width. Samples were prepared as 10.16 cm (4 inch) by 10.16 cm (4 inch) test specimens with edges parallel to the machine direction and transverse direction for substrates with directionality. Three rigidity measurements were determined on the same side of fresh test specimens orientated in the same substrate direction. A further three rigidity measurements were taken on the same side of fresh test specimens oriented at 90° to the first orientation. These six measurements were repeated on the opposite side to the first six measurements, on fresh test samples. The 12 rigidity measurements were then averaged and reported to 0.01 g/cm.

The rigidity of a substrate is a function of substrate thickness and inherent modulus of elasticity. Different materials have different moduli of elasticity. Based upon the material or materials that the substrate comprises, a substrate thickness should be selected that enables the desired rigidity of the substrate to be achieved.

The water impermeable substrate preferably has a thickness of from 80 µm to 12 µm, more preferably from 50 µm to 15 µm, even more preferably from 40 µm to 16 µm and even more preferably still from 30 µm to 17 µm.

Non-limiting examples of substrate material and thickness combinations for the water impermeable substrate are:

| **Substrate Material** | **Thickness [microns]** | **Rigidity [g/cm]** |
|---|---|---|
| HDPE | 13 | 0.13 |
| HDPE | 18 | 0.33 |
| HDPE | 36 | 1.05 |
| LLDPE | 23 | 0.23 |
| PP | 18 | 0.46 |

| | | |
|---|---|---|
| [HDPE is a mixture of LBI 85% M6030 and Exxon Mobil 15% LD2001 manufactured on a Merritt-Davis casting line] [LLDPE is Exxon Mobil 15% LD2001 manufactured on a Merritt-Davis casting line] [PP is Basell PH835 manufactured on a Merritt-Davis casting line] | | |

The water impermeable substrate may comprise a textured or, alternatively, micro-structured surface on at least a portion of one side. Surface texturing or micro-structuring increases the effective surface area of the water impermeable substrate and thus improves adherence of the aqueous depilatory composition to said water impermeable substrate, facilitating an easy removal of the depilatory article by peeling it off the skin, or increases the grip of the surface, thus improving handleability. The textured structures may comprise dimples; lines or curvilinear embossments. A textured surface may be formed on the substrate by any appropriate technique, including embossment calendars and casting.

The water impermeable substrate may be manufactured by any suitable method, including casting, injection moulding, co-injection moulding, over moulding, in-mold assembly, compression moulding, blow moulding, casting thermo or vacuum forming.

Achieving a desired dosage of depilatory composition to the surface of the skin is a further advantage of using a substrate-based product. However, if the substrate is able to stretch or tear, the layer of depilatory composition disposed upon it may be thinned, thickened or rupture in places, resulting in uneven and hence less desirable depilatory activity. In particular, low depilatory efficacy may result in areas treated with thinned or ruptured areas of the composition while higher depilatory efficacy and increased irritation may result in areas treated with thickened areas of the composition.

The potential problem of a substrate stretching may be avoided by selecting a substrate that does not permanently deform during use. This problem may also be avoided by selecting a substrate with a sufficiently high secant modulus such that it is less likely to stretch during normal use. Accordingly, in another preferred embodiment, the water impermeable substrate has a secant modulus at 2% strain of greater than 689.5 bar (10,000 psi), more preferably greater than 1379.0 bar (20,000 psi), even more preferably greater than 2068.4 bar (30,000 psi) and even more preferably still greater than 2757.9 bar (40,000 psi) in order to achieve uniform application of the aqueous depilatory composition to the surface of the body during usage. Without wishing to be bound by theory, applicants believe that using a substrate with an excessively low secant modulus at 2% strain can deform and thus break apart the aqueous depilatory composition disposed on the substrate, leading to uneven depilatory action and increased risk of irritation. The secant modulus at 2% strain may be measured readily using the American Standard Test Method (ASTM)) 'Standard Test Method for Tensile Properties of Thin Plastic Sheeting D882-09' conducted on an MTS Insight1 Tensile Tester available from MTS Systems Co, Eden Prairie, MN, USA. This method may also be applied to non-plastic materials and is designed for use on sheets with a thickness of less than I mm.

The potential problem of a substrate tearing may be avoided by selecting a substrate that does not fail during usage. This problem may also be avoided by selecting a substrate with a sufficiently high nominal tensile strength such that it is less likely to tear during normal use. Accordingly, in another preferred embodiment, the water impermeable substrate has a nominal tensile strength of at least 5 MPa more preferably at least 10 MPa even more preferably at least 15 MPa and even more preferably still at least 18 MPa in order to achieve uniform application of the aqueous depilatory composition to the surface of the body during usage. Without wishing to be bound by theory, applicants believe that using a substrate with an excessively low nominal tensile strength can fail during usage and thus break apart the aqueous depilatory composition disposed on the substrate, leading to uneven depilatory action and increased risk of irritation. The nominal tensile strength may be measured readily using the American Standard Test Method (ASTM) 'Standard Test Method for Tensile Properties of Thin Plastic Sheeting D882-09' conducted on an MTS Insight1 Tensile Tester available from MTS Systems Co, Eden Prairie, MN, USA. This method may also be applied to non-plastic materials and is designed for use on sheets with a thickness of less than 1 mm.

A layer of aqueous depilatory composition can be applied to the water impermeable substrate through any known technique of applying viscous fluids to substrates, including, for example, extrusion, casting (e.g., reverse roll, knife-over roll, slot die, Gravure roll), spraying, knife blade coating, and zone coating. Such techniques may be modified to alter the quantity of aqueous depilatory composition disposed on the water impermeable substrate. For example, the speed at which the water impermeable substrate travels through an extrusion process determines the quantity of aqueous depilatory composition disposed upon said water impermeable substrate. The area of aqueous depilatory composition may cover the entire surface of the water impermeable substrate of a portion thereof. Advantageously, the aqueous depilatory composition covers less than the entire surface of the water impermeable substrate to facilitate handling. The water impermeable substrate may comprise at least one region with two orthogonal dimensions each of a length greater than 1 cm, preferably greater than 1.5 cm and more preferably greater than 2 cm upon which no aqueous depilatory composition is disposed.

Preferably, the aqueous depilatory composition is disposed upon the water impermeable substrate in an amount per unit area of 0.300 g/cm² to 0.001 g/cm², more preferably from 0.015 g/cm² to 0.003 g/cm², even more preferably from 0.080 g/cm² to 0.005 g/cm² and even more preferably still from 0.05 g/cm² to 0.005 glcm², wherein the unit area refers to the coated region of the water impermeable substrate and not including any uncoated surface of the water impermeable substrate. Additionally, the area used to calculate the amount of aqueous depilatory composition disposed upon the water impermeable substrate is calculated ignores any surface texturing or micro-structuring. Alternatively, the mean thickness of the aqueous depilatory composition is preferably from 0.01 mm to 3 mm, more preferably 0.1 mm to 1.5 mm, even more preferably from 0.05 mm to 0.8 mm, and even more preferably still from 0.05 mm to 0.5 mm.

The concentration of water in the aqueous depilatory composition is preferably at least 40%, more preferably from 50% to 98%, even more preferably from 60% to 95% and even more preferably still from 70% to 90%, by weight of the aqueous depilatory composition. This high water level helps to improve the overall skin mildness of the aqueous depilatory composition by being dilute, and to keep the system more robust to pH changes, which may result in skin irritation.

The aqueous depilatory composition may optionally comprise a thickening agent. A representative but not exhaustive list can be found in "The Encyclopaedia of Polymers and Thickeners for Cosmetics" compiled and edited by Robert Y. Lochhead, PhD and William R. Fron, Department of Polymer Science, University of Southern Mississippi. Exemplary classes of thickening agents include gums, carbomers, polymers and copolymers of acrylic acid, associated thickeners, layered silicates/clays and natural polymers (including polysaccharides). One or more thickening agents may be included in the aqueous depilatory composition. It may be desirable to utilize gel network structures or oil-in-water emulsions to thicken the aqueous depilatory compositions. Suitable materials for preparing the gel network structures or oil-in-water emulsions are well represented in the art and include fatty materials such as fatty alcohols (for example cetyl alcohol and stearyl alcohol) alone or used in conjunction with non-polar oils such as paraffin or mineral oils. An appropriate emulsifier may also be used to form and stabilize the bilayer structure characteristic of gel network structures or to form and stabilize an oil-in-water emulsion. The thickening agent may be present at a level of from about 0.01% to about 20%, preferably from about 0.1% to about 10%, more preferably from about 0.3% to about 5%, and even more preferably from about 0.5% to about 4%, by weight of the aqueous depilatory composition.

Advantageously, the thickening agent comprises carrageenan. The carrageenan is preferably present in an amount of from 0.1% to 10%, more preferably from 0.5% to 8%, even more preferably from 1% to 5% and even more preferably still from 2% to 4% by weight of the aqueous depilatory composition. The carrageenan may be iota, kappa or lambda carrageenan, and in a preferred embodiment is iota carrageenan. Without wishing to be bound by theory, the applicants believe that a depilatory composition comprising carrageenan has both an affinity to the surface of the skin, providing an effect analogous to a frictional resistance opposing spreading of the composition and cohesive forces that further prevent spreading and additionally prevent rupturing of the composition.

The rheological properties of the aqueous depilatory composition may also lead to improved performance in use. In particular, the yield point describes the resistance of the aqueous depilatory composition to deformation under environmental stress. If the yield point is too high, then the aqueous depilatory composition may not deform sufficiently, with hair fibres unable to enter the aqueous depilatory composition effectively upon application, resulting in less desirable depilatory effectiveness. If the yield point is too low, however, then the aqueous depilatory composition may flow during storage, transport or use and is not cleanly removed from the skin upon removal of the depilatory article, thus requiring the inconvenience of additional wiping and risking irritation to the user. Accordingly, the phase angle of the aqueous depilatory composition preferably has a yield point from 10 Pa to 2000 Pa, more preferably from 30 Pa to 1200 Pa, even more preferably from 45 Pa to 500 Pa and even more preferably still from 60 Pa to 300 Pa, when measured via a stress controlled amplitude sweep at a frequency of 1Hz and a temperature of 25°C. The yield point described is defined as the 5% decrease in magnitude of the elastic modulus G' linear viscoelastic plateau value as measured on a TA 1000 Rheometer, available from TA Instruments of New Castle, Delaware, USA. The rheological properties of the aqueous depilatory composition may be altered by changing the concentration or identity of the thickening system and the water content of the aqueous depilatory composition.

Advantageously, the aqueous depilatory composition displays an elastic modulus G' which exceeds its viscous modulus G" at all frequencies below 60 rad/s, preferably below 20 rad/s, more preferably below 10 rad/s and even more preferably below 1 rad/s; when measured via a strain controlled frequency sweep; at a strain of 1% and a temperature of 25°C. The elastic modulus of the aqueous depilatory composition exceeds its viscous modulus at a low frequency of applied stress. This indicates that the aqueous depilatory composition is behaving in a Solid-like manner at rest and is of particular benefit when the aqueous depilatory composition is interposed between two substrates, for example a substrate and a protective release layer.

In another preferred embodiment, the aqueous depilatory composition displays a high degree of shear thinning behaviour enabling the effective coating of target hairs during application and improve depilatory efficacy. Accordingly, at a low shear rate of 0.1 s⁻¹, the dynamic viscosity of the aqueous depilatory composition is preferably 1000 Pa.s to 10000 Pa.s measured at a temperature of 25°C, whereas at a high shear rate of 1000 s⁻¹, the dynamic viscosity of the aqueous depilatory composition is preferably 0.1 Pa.s to 1 Pa.s, measured at a temperature of 25°C.

The aqueous depilatory composition may also include other skin care ingredients such as conditioning agents selected from the group consisting of humectants, moisturizers, or skin conditioners (including mineral oil; almond oil; chamomile oil; jojoba oil; avocado oil; shea butter, niacinamide and glycerine); skin rejuvenation compositions (for example targeted for fine lines, wrinkles and uneven skin tone including retinoids), cosmetic compositions; antiinflammatory agents (including corticosteroids); anti-oxidants (including flavonoids) radical scavengers; sunscreen agents; skin cooling or warming agents and the like. The aqueous depilatory composition may comprise one or more skin care ingredients present in an amount of from about 0.001% to about 10%, more preferably from about 0.01% to about 7%, and even more preferably from about 0.025% to about 5%, by weight of the aqueous depilatory composition.

An accelerant may be employed in the aqueous depilatory composition. This optional component accelerates the rate of depilatory action of the depilatory agent. Suitable accelerants include, but are not limited to, urea; thiourea; dimethyl isosorbide; arginine salts; ethoxydiglycol; propylene glycol and methylpropyldiol. The accelerant may be present in a concentration range of from 0.5% to 10%, more preferably from 2% to 8% and even more preferably from 2% to 5% by weight of the aqueous depilatory composition.

The aqueous depilatory composition may further comprise components known, conventionally used, or otherwise effective for use in hair removal compositions particularly dyes; pigments (including ultra marines and talc); anionic, cationic, non-ionic and/or amphoteric or zwitterionic surfactants, polymers (including hydrophobically modified polymers); dispersing agents; solvents; lubricants; fragrances; preservatives; chelants, proteins and derivatives thereof, plant materials (e.g. aloe, chamomile and henna extracts); silicones (volatile or non-volatile, modified or non-modified); film-forming agents; film forming promoters and mixtures thereof.

Depilatory articles of the present invention may take any form suitable for applying to keratinous tissue. The size and shape of the depilatory article may take any form suitable for application to the body area from which hair is to be removed. The depilatory article will preferably relate to the body area or zone from which hair is to be removed, especially the face (including the jaw, chin and upper lip regions of the face), underarm and bikini areas. Preferably, the depilatory article takes the form of a mask (configured for the face) or a strip/patch (configured for general use). In another preferred embodiment, the water impermeable substrate of the depilatory article is substantially planar.

The coated region preferably comprises an upper-lip portion adapted to be placed above a human mouth, and a first return portion projecting from the upper lip portion and adapted to be placed contiguously with the outer extremity of the vermilion lip in a first corner of the mouth. The return portion has a length along its greatest dimension of at least 0.2 cm, preferably from 0.5 cm to 5 cm, more preferably from 0.75 cm to 4 cm, even more preferably from 1 cm to 3 cm. Applicants have found that this configuration enables the user to remove unwanted hair from the skin immediately surrounding the corner of the mouth while lowering the risk of depilatory composition contacting the vermillion lip, where it may cause irritation. In an alternative embodiment, the coated region further comprises a second return portion projecting from the upper lip portion and adapted to be placed contiguously with the outer extremity of the vermillion lip in a second corner of the mouth.

Advantageously, the upper lip portion has a length along its greatest dimension of at least 0.2 cm, preferably from 0.5 cm to 15 cm, more preferably from 1 cm to 12 cm, even more preferably from 2 cm to 10 cm and even more preferably still from 3 cm to 8 cm. This dimension enables the upper lip portion to cover a desirable length of the upper lip and thus achieve the desired depilatory action. In a preferred embodiment, the upper lip portion is adapted to be placed to be at least partially contiguously with the upper border of the upper vermilion lip, to enable depilatory action to be achieved on the skin immediately surrounding the upper vermilion lip while lowering the risk of depilatory composition contacting, the upper vermilion lip, where it may cause irritation.

In another preferred embodiment, the coated region comprises a lower lip portion adapted to be placed below a human mouth, preferably wherein the lower lip portion is adapted to be placed to be least partially contiguously with the lower border of the lower vermilion lip to enable depilatory action to be achieved on the skin immediately surrounding the lower vermilion lip while lowering the risk of depilatory composition contacting the lower vermilion lip, where it may cause irritation.

Depilatory articles of the present invention may comprise at least two finger-tabs being substantially free of depilatory composition and positioned on substantially opposing sides of the coated region. These finger tabs enable a user to apply tension to the coated region of the substrate. Surprisingly, applicants have found that applying tension across the coated region of the depilatory article creates an effect of temporarily causing the coated region to exhibit an apparent increased rigidity, enabling the user to accurately position the coated region, and hence depilatory composition on to the desired region of the body. Tensioning the coated region may be achieved in a number of ways, non-limiting examples of which include holding the depilatory article either side of the coated region, for example with the hands or a tool, so as to apply tension between the areas being held. Alternatively, depilatory articles of the present invention may comprise at least one finger-tab being substantially free of depilatory composition and positioned to allow the weight of the article to tension the coated region when being held by the finger-tab.

In a preferred embodiment, at least one finger tab extends from the perimeter of the coated region by a minimum of 1 cm, preferably from 1.5 cm to 5 cm, more preferably from 2 cm to 4 cm and even more preferably from 2.5 cm to 3.5 cm. In another preferred embodiment, both finger-tabs extend from the perimeter of the coated region by a minimum of 1 cm, preferably from 1.5 cm to 5 cm, more preferably from 2 cm to 4 cm and even more preferably from 2.5 cm to 3.5 cm, in order to aid handling of the depilatory article.

Depilatory articles of the present invention may comprise a protective release layer removably attached to the aqueous depilatory composition, preferably on a surface of the aqueous depilatory composition substantially opposing that which is in contact with the substrate. The protective release layer may comprise materials including polymer resins such as a polyolefins e.g. polypropylene (including stratified biaxially oriented polypropylene (SBOPP)), polyethylene (including LDPE; LLDPE; HDPE; Metallocene) or polyethylene terephthalate. Alternative materials which may be used include polyvinylchloride, polyamide, acetyl, acrylonitrile butadiene styrene, acrylic, acrylonitrile styrene acrylate, ethylene vinyl alcohol, ethylene vinyl acetate, Nylon, Latex, natural or synthetic rubbers, polycarbonate, polystyrene, silicone or thermo plastic elastomer, thermo plastic vulcanate or copolymers of said materials. Where appropriate the protective release layer may comprise one or more laminations, combinations of multiple layers and/or indications (which may include instructions and illustrations) relating to at least one aspect of the usage of the depilatory article. In an advantageous the protective release layer may comprise a coating of a non-stick material. Exemplary non-stick coatings include wax, silicone, fluoropolymers such as TEFLON®, and fluorosilicones. In a preferred embodiment, the protective release layer covers at least the entire aforementioned coated region of the substrate. In another preferred embodiment the protective release layer is water impermeable. In a further preferred embodiment, the protective release layer has a mean thickness of at least 85 microns, more preferably from 85 microns to 130 microns, even more preferably from 90 microns to 120 microns. In yet another preferred embodiment, the protective release layer extends beyond the coated region of the substrate to provide a removal tab.

In a preferred embodiment, the depilatory articles of the present invention are packaged to prevent water loss and/or oxygen permeation. Alternatively, the depilatory articles of the present invention are packaged in water impermeable packaging. Examples of suitable packaging materials include films of EVOH; PP; PE; Nylon; or mixtures thereof. More preferably, the packaging comprises an inert gas and even more preferably the inert gas comprises at least one of nitrogen, argon or carbon dioxide. Alternatively, the packaging comprises a partial vacuum.

A second aspect being a method of removing hair from the skin is also provided by the present invention, comprising the steps of:
(a) applying a depilatory article according to the present invention to the surface of the skin, preferably mammalian and more preferably human skin,
(b) leaving said depilatory article in contact with the skin for a period of at least 1 minute, preferably 2 to 10 minutes, more preferably 2 to 8 minutes,
(c) removing said depilatory article from the surface of the skin, and
(d) preferably rubbing, scraping, rinsing or wiping the surface of the skin in the area to which the depilatory article was applied.

Advantageously, the method of removing hair from the skin further comprises the step of tensioning the coated region of the depilatory article prior to applying it to the skin.

The same means used to apply tension to the coated region may be used to ensure that the depilatory article is applied to the surface of the body such that the coated region is applied under tension to the unwanted hair in order to maintain the improved handling characteristics described above. In a preferred embodiment, the tension is kept substantially constant during application of the depilatory article. The flexible nature of the substrate allows the substrate to conform to the surface of the body to offer improved contact between the aqueous depilatory composition and the unwanted hair. In a preferred embodiment, the tension may be at least partially, more preferably substantially completely released from the coated region after applying the depilatory article to the skin in order to improve the conformability of the depilatory article.

A third aspect being a depilatory kit is also provided by the present invention, which comprises at least one depilatory article of the present invention, packaging for said depilatory article(s), and at least one of a third component selected from:
a) a pre-treatment skin care composition which may comprise ingredients to promote skin conditioning (e.g. emollients), hair hydration or provide a skin barrier (e.g. hydrophobic materials) and intended for use prior to applying the depilatory article.
b) a post-treatment skin care composition which may comprise ingredients to promote skin conditioning; moisturizers, skin rejuvenation compositions (targeted for fine lines, wrinkles and uneven skin tone, for example), cosmetic compositions (e.g., foundation, rouge), sunscreens and the like as described herein above. The complementary post treatment skin care compositions may be leave-on or rinse-off compositions. The skin care compositions may also be designed to immediately follow application of the hair removal products. For example, a finishing composition may be applied to the same skin area to combat lingering odour and irritation caused by residual depilatory agent. The finishing composition may comprise a metal oxide (e.g., zinc oxide, aluminum oxide, and magnesium oxide) that is capable of complexing with any remaining depilatory agent remaining on the targeted skin area to reduce continued odour and subsequent skin irritation.
c) a tool to assist in the removal of hair and/or aqueous depilatory composition from the skin.
d) indications (which may include instructions and/or illustrations) relating to at least one aspect of usage of the depilatory article or another component of the kit.

Reference is made to the figures, which disclose a non-limiting embodiment of the invention.

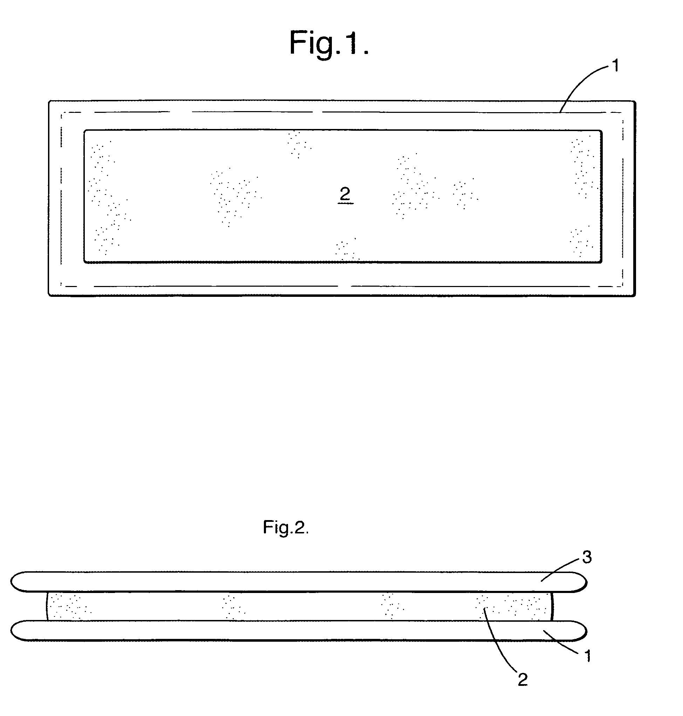

Fig.1 depicts a plan view of a depilatory article of the present invention, comprising a water impermeable substrate(1) and an aqueous depilatory composition(2). Fig. 2 depicts a side view of a depilatory article of the present invention, further comprising a protective release layer(3).

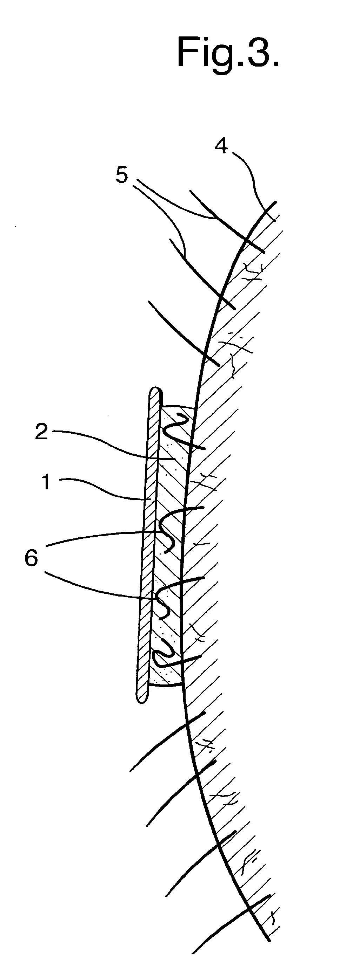

Fig.3 depicts a side view of a depilatory article of the present invention in use, i.e. applied to keratinous tissue which comprises the skin(4), hair strands outside the aqueous depilatory composition(5) and hair strands within the aqueous depilatory composition(6).

### Example

The following examples further describe and demonstrate one embodiment within the scope of the present invention. The examples are given solely for the purpose of illustration and are not to be construed as a limitation of the present invention, as many variations thereof are possible.

Comparative Example:

| Comparative Formulation Ingredients | % w/w |
|---|---|
| DI water | 85.00 |
| Acrylic Acid / VP Crosspolymer (Ultrathix P-100)¹ | 4.50 |
| Calcium Hydroxide² | 4.50 |
| Calcium Thioglycolate Trihydrate³ | 6.00 |

| | |
|---|---|
| 1 Ultrathix P-100 available from International Specialty Products Inc. (ISP) 2 Calcium Hydroxide Reag. Ph. Eur. puriss. p.a. available from Sigma-Aldrich Co. 3 Calcium Thoglycolate Trihydrate 99.8% available from BRUNO BOCK Chemische Fabrik GmbH & Co. | |

A 400 ml speed mixer plastic pot was sanitized and DI water weighed in directly. The calcium hydroxide was added with mixing and the batch was then heated to 37 °C in a water bath for 10 minutes. The Ultrathix P-100 was then slowly added to the batch in portions over 7 minutes (increasing the mixing speed if required). The batch was mixed for a further 10 minutes (again, increasing the mixing speed as required). The batch was then cooled to room temperature using a water jacket and the calcium thioglycolate slowly added. After a further 10 minutes to ensure full incorporation of the calcium thioglycolate and batch homogeneity the batch was then transferred to a thick walled 400 ml glass beaker and milled for 2 minutes using an IKA T50 (5,200 rpm).

The comparative formulation was disposed to a thickness of 0.3 mm over an area of 3.0 x 3.5 cm on a cast HDPE 85% LLDPE 15% polymer blend film (31 microns in thickness, 4.6 cm in length and 3.2 cm in width) using a stencil and wiper blade, such that the area covered by the comparative formulation was centered along the width of the film and I mm away from the perimeter edge of one end of the film's length.

Inventive Example:

| Inventive Formulation Ingredient | % w/w |
|---|---|
| DI water | 84.42 |
| Acrylic Acid / VP Crosspolymer (Ultrathix P-100)¹ | 3.00 |
| Sodium Silicate (42% w/w in water) (Cognis 60)⁴ | 2.08 |
| Calcium Hydroxide² | 4.50 |
| Calcium Thioglycolate Trihydrate³ | 6.00 |

| | |
|---|---|
| 1 Ultrathix P-100 available from International Specialty Products Inc. (ISP) 2 Calcium Hydroxide Reag. Ph. Eur. puriss. p.a. available from Sigma-Aldrich Co. 3 Calcium Thoglycolate Trihydrate 99.8% available from BRUNO BOCK Chemische Fabrik GmbH & Co. 4 Sodium Silicate (Cognis 60) available from Cognis | |

A 400 ml speed mixer plastic pot was sanitized and DI water weighed in directly. The calcium hydroxide was added with mixing and the batch was then heated to 37 °C in a water bath for 10 minutes. The Ultrathix P-100 was then slowly added to the batch in portions over 7 minutes (increasing the mixing speed if required). The batch was mixed for a further 10 minutes (again, increasing the mixing speed as required). The batch was then cooled to room temperature using a water jacket and the sodium silicate was added slowly followed by the calcium thioglycolate. After a further 10 minutes to ensure full incorporation of the calcium thioglycolate and batch homogeneity the batch was then transferred to a thick walled 400 ml glass beaker and milled for 2 minutes using an IKA T50 (5,200 rpm). The amount of Ultrathix P-100 (thickener) used in the inventive example was less than that used in the comparative example to achieve comparable rheological properties and remove this as a potential cause for different results.

The inventive formulation was disposed to a thickness of 0.3 mm, width of 3.0 cm and length of 3.5 cm on a cast HDPE 85% LLDPE 15% polymer blend film (31 microns in thickness, 4.6 cm in length and 3.2 cm in width) using a stencil and wiper blade, such that the area covered by the inventive formulation was centered along the width of the film and I mm away from the perimeter edge of one end of the film's length.

The comparative and inventive examples were evaluated for the amount of hair removed on the outer forearm of 17 male panelists in a paired study. The identity of the examples was unknown to the panelists in the study. The examples were coded A for the inventive example and B for the comparative example.

A test area of 3.0 × 3.5 cm was clearly marked on the outer forearm with a skin pen and labeled A for the inventive example. A second test area of 3.0 × 3.5 cm was also marked on the same outer forearm of the same panelist and labeled B for the comparative example. The two test areas were selected such that both areas had the same degree of hair and were separated by a distance of 1.0 cm. The comparative example B was placed on test area B and gently dabbed to ensure contact of the formulation with the hair and skin. After 3 minutes the comparative example B was removed by the panelist and the area wiped with tissue to remove the formulation and hair. The inventive example A was placed on the other test area and gently dabbed to ensure contact of the formulation with the hair and skin. After 3 min the inventive example A was removed by the panelist and the area wiped with tissue to remove the formulation and hair. The order of application of the examples and location on the male forearm was randomized for each panelist.

On completion each panelist was asked the following questions:
1) Is there a difference in the amount of hair removed in test area A compared to test area B?
2) Which test area had the most hair removed?

Results: Panelists' responses to questions:

| **Question** | **Number of Panellist's for each response** | | | |
|---|---|---|---|---|
| | **Yes** | | **No** | |
| Is there a difference in the amount of hair removed in test area A compared to test area B? | 17 s | | 0 | |
| | | | | |

| **Question** | **Number of Panellist's for each response** | | | |
|---|---|---|---|---|
| | **A** | **B** | | **No difference** |
| Which test area had the most hair removed? | 15 s | 2 | | 0 |

Results were analyzed with statistical paired t-test. s denotes a statistical difference (at a level of significance of 0.2).

The consumer results show that the inventive example A results in more hair removal than the comparative example B.

## Claims

1. A depilatory article comprising:
(a) a water impermeable substrate (1); and
(b) an aqueous depilatory composition (2), the aqueous depilatory composition being in physical contact with the water impermeable substrate (1), forming a coated region of the water impermeable substrate(1) and comprising:
i. a thioglycolate salt;
ii. a water-soluble silicate;
iii. a divalent metal cation; and
iv. a monovalent cation.

2. A depilatory article according to claim 1 wherein the water-soluble silicate is present in the aqueous depilatory composition in an amount per unit area of said coated region of the water impermeable substrate of from 2.05 × 10⁻⁸ mol/cm⁻² to 1.23 × 10⁴ mol/cm², preferably from 1.64 × 10⁻⁷ mol/cm² to 3.69 × 10⁻⁵ mol/cm² and more preferably from 4.92 × 10⁻⁷ mol/cm² to 8.20 × 10⁻⁶ mol/cm².

3. A depilatory article according to either preceding claim, wherein the aqueous depilatory composition comprises a base, preferably a buffering base and preferably in a concentration range of from 0.1% to 10.0%, more preferably from 0.5% to 8% and even more preferably from 1% to 5% by weight of the aqueous depilatory composition.

4. A depilatory article according to claim 3, wherein the base comprises a divalent cation, preferably a divalent metal cation, more preferably a calcium cation, magnesium cation, zinc cation or mixtures thereof and even more preferably a calcium cation.

5. A depilatory article according to any preceding claim, wherein the thioglycolate salt comprises a divalent cation, preferably a divalent metal cation, more preferably a divalent zinc, calcium, magnesium, barium or strontium cation, or mixtures thereof and even more preferably a calcium cation.

6. A depilatory article according to any preceding claim, wherein the concentration of the conjugate acid of the thioglycolate salt is from 0.5% to 12.0%, more preferably from 0.8% to 8.0% and even more preferably from 1.0% to 6.0% by weight of the aqueous depilatory composition.

7. A depilatory article according to any preceding claim, wherein the aqueous depilatory composition comprises monovalent cations in amount per unit area of the coated region of the water impermeable substrate of less than 5.10 × 10⁻⁴ mol/cm², preferably less than 3 × 10⁻⁴ mol/cm⁻², more preferably from 1 × 10⁻⁹ mol/cm² to 1.5 × 10⁻⁴ mol/cm², even more preferably from 2.50 × 10⁻⁸ mol/cm² to 6.65 × 10⁻⁵ mol/cm² and even more preferably still from 6 × 10⁻⁷ mol/cm² to 4.5 × 10⁻⁵ mol/cm².

8. A depilatory article according to any preceding claim, wherein the aqueous depilatory composition comprises a water-soluble silicate selected from: lithium silicates, sodium silicates (including disodium metasilicate pentahydrate and disodium metasilicate nanohydrate), potassium silicates, ammonium silicates, imidazolium silicates, synthetic silicates (clays) or mixtures thereof; more preferably selected from: synthetic clays, sodium silicates, potassium silicates, or mixtures thereof and even more preferably wherein the aqueous depilatory composition comprises a sodium silicate or mixtures of sodium silicates.

9. A depilatory article according to any preceding claim, wherein wherein the aqueous depilatory composition comprises water in an amount of at least 40%, preferably from 50% to 98%, more preferably from 60% to 95% and even more preferably from 70% to 90%, by weight of the aqueous depilatory composition.

10. A depilatory article according to any preceding claim, wherein the depilatory article is a mask, strip or patch.

11. A depilatory article according to any preceding claim, wherein said water impermeable substrate comprises a water impermeable material, preferably a plastic sheet, more preferably a polyolefin, even more preferably a polyethylene and even more preferably still high density polyethylene.

12. The depilatory article according to any preceding claim, wherein a protective release layer (3) is removably attached to the aqueous depilatory composition.

13. A method of removing hair from the skin, comprising the steps of:
(a) applying a depilatory article according to any preceding claim to a surface of skin, preferably human skin,
(b) leaving said depilatory article in contact with the skin for a period of greater than 1 minute, preferably 2 to 10 minutes, more preferably 2 to 8 minutes
(c) removing said depilatory article from the surface of the skin, and
(d) preferably rubbing, scraping, rinsing or wiping the surface of the skin in the area to which the depilatory article was applied.

14. A depilatory kit, comprising:
(a) at least one depilatory article according to any of claims 1-12,
(b) optionally, at least one of a pre-treatment skin care composition, a post-treatment skin care composition and/or a tool to assist removal of hair and/or aqueous depilatory composition after use, and
(c) Packaging for said depilatory kit.

## Patentansprüche

1. Depilierartikel, der Folgendes umfasst:
(a) ein wasserundurchlässiges Substrat (1); und
(b) eine wässrige Depilierzusammensetzung (2), wobei die wässrige Depilierzusammensetzung in physischem Kontakt mit dem wasserundurchlässigen Substrat (1) steht, dabei einen beschichteten Bereich an wasserundurchlässigem Substrat (1) bildet und Folgendes umfasst:
i. ein Thioglycolatsalz;
ii. ein wasserlösliches Silikat;
iii. ein zweiwertiges Metallkation; und
iv. ein einwertiges Kation.

2. Depilierartikel nach Anspruch 1, wobei das wasserlösliche Silikat in der wässrigen Depilierzusammensetzung in einer Menge pro Einheitsfläche an wasserundurchlässigem Substrat von 2,05 x 10⁻⁸ mol/cm⁻² bis 1,23 x 10⁻⁴ mol/cm² vorliegt, vorzugsweise von 1,64x 10⁻⁷ mol/cm² bis 3,69 x 10⁻⁵ mol/cm² und mehr bevorzugt von 4,92 x 10⁻⁷ mol/cm² bis 8,20 x 10⁻⁶ mol/cm².

3. Depilierartikel nach einem der vorgenannten Ansprüche, wobei die wässrige Depilierzusammensetzung eine Basis umfasst, vorzugsweise eine Pufferbasis und vorzugsweise in einer Konzentration von 0,1 Gew.-% bis 10,0 Gew.-%, mehr bevorzugt von 0,5 Gew.-% bis 8 Gew.-% und noch mehr bevorzugt von 1 Gew.-% bis 5 Gew.-% der wässrigen Depilierzusammensetzung.

4. Depilierartikel nach Anspruch 3, wobei die Basis ein zweiwertiges Kation umfasst, vorzugsweise ein zweiwertiges Metallkation, mehr bevorzugt ein Kalziumkation, Magnesiumkation, Zinkkation oder Mischungen davon und noch mehr bevorzugt ein Kalziumkation.

5. Depilierartikel nach einem der vorgenannten Ansprüche, wobei das Thioglycolatsalz ein zweiwertiges Kation umfasst, vorzugsweise ein zweiwertiges Metallkation, mehr bevorzugt ein zweiwertiges Zink-, Kalzium-, Magnesium-, Barium- oder Strontiumkation, oder Mischungen davon und noch mehr bevorzugt ein Kalziumkation.

6. Depilierartikel nach einem der vorgenannten Ansprüche, wobei die Konzentration an Konjugatsäure des Thioglycolatsalzes von 0,5 Gew.-% bis 12,0 Gew.-%, mehr bevorzugt von 0,8 Gew.-% bis 8,0 Gew.-% und noch mehr bevorzugt von 1,0 Gew.-% bis 6,0 Gew.-% der wässrigen Depilierzusammensetzung beträgt.

7. Depilierartikel nach einem der vorgenannten Ansprüche, wobei die wässrige Depilierzusammensetzung einwertige Kationen in einer Menge pro Einheitsfläche an beschichtetem Bereich des wasserundurchlässigen Substrats von weniger als 5,10 x 10⁻⁴ mol/cm² umfasst, vorzugsweise weniger als 3 x 10⁻⁴ mol/cm⁻², mehr bevorzugt von 1 x 10⁻⁹ mol/cm² bis 1,5 x 10⁻⁴ mol/cm², noch mehr bevorzugt von 2,50 x 10⁻⁸ mol/cm² bis 6,65 x 10⁻⁵ mol/cm² und noch mehr bevorzugt von 6 x 10⁻⁷ mol/cm² bis 4,5 x 10⁻⁵ mol/cm².

8. Depilierartikel nach einem der vorgenannten Ansprüche, wobei die wässrige Depilierzusammensetzung ein wasserlösliches Silikat umfasst, ausgewählt aus: Lithiumsilikaten, Natriumsilikaten (einschließlich Dinatriummetasilikatpentahydrat und Dinatriummetasilikatnanohydrat), Kaliumsilikaten, Ammoniumsilikaten, Imidazoliumsilikaten, synthetischen Silikaten (Tonerden) oder Mischungen davon; mehr bevorzugt ausgewählt aus: synthetischen Tonerden, Natriumsilikaten, Kaliumsilikaten oder Mischungen davon, und wobei die wässrige Depilierzusammensetzung noch mehr bevorzugt ein Natriumsilikat oder Mischungen aus Natriumsilikaten umfasst.

9. Depilierartikel nach einem der vorgenannten Ansprüche, wobei die wässrige Depilierzusammensetzung Wasser in einer Menge von mindestens 40 Gew.-%, vorzugsweise von 50 Gew.-% bis 98 Gew.-%, mehr bevorzugt von 60 Gew.-% bis 95 Gew.-% und noch mehr bevorzugt von 70 Gew.-% bis 90 Gew.-% der wässrigen Depilierzusammensetzung umfasst.

10. Depilierartikel nach einem der vorgenannten Ansprüche, wobei der Depilierartikel eine Maske, ein Streifen oder ein Pflaster ist.

11. Depilierartikel nach einem der vorgenannten Ansprüche, wobei das wasserundurchlässige Substrat ein wasserundurchlässiges Material umfasst, vorzugswiese eine Kunststofffolie, mehr bevorzugt ein Polyolefin, noch mehr bevorzugt ein Polyethylen und noch mehr bevorzugt Niederdruckpolyethylen.

12. Depilierartikel nach einem der vorgenannten Ansprüche, wobei eine abziehbare Schutzschicht (3) ablösbar an der wässrigen Depilierzusammensetzung angebracht ist.

13. Verfahren zum Entfernen von Haaren von Haut, , das folgende Schritte umfasst:
(a) Anwenden eines Depilierartikels nach einem der vorgenannten Ansprüche auf eine Hautoberfläche, vorzugsweise auf menschliche Haut,
(b) Belassen des Depilierartikels in Kontakt mit der Haut über einen Zeitraum von mehr als 1 Minute, vorzugsweise 2 bis 10 Minuten, mehr bevorzugt 2 bis 8 Minuten
(c) Abziehen des Depilierartikels von der Hautoberfläche, und
(d) vorzugsweise Abreiben, Abschaben, Abspülen oder Abwischen der Hautoberfläche in dem Bereich, auf den der Depilierartikel angewandt wurde.

14. Depilierset, das Folgendes umfasst:
(a) mindestens einen Depilierartikel nach einem der Ansprüche 1-12,
(b) wahlweise mindestens eine Hautvorbehandlungszusammensetzung, eine Hautnachbehandlungszusammensetzung und/oder ein Instrument zum besseren Entfernen von Haar und/oder der wässrigen Depilierzusammensetzung nach Gebrauch, und
(c) Verpackung für das Depilierset.

## Revendications

1. Article dépilatoire comprenant :
(a) un substrat imperméable à l'eau (1) ; et
(b) une composition dépilatoire aqueuse (2), la composition dépilatoire aqueuse étant en contact physique avec le substrat imperméable à l'eau (1), formant une région revêtue du substrat imperméable à l'eau (1) et comprenant :
i. un sel thioglycolate ;
ii. un silicate hydrosoluble ;
iii. un cation métallique divalent ; et
iv. un cation monovalent.

2. Article dépilatoire selon la revendication 1, dans lequel le silicate hydrosoluble est présent dans la composition dépilatoire aqueuse en une quantité par surface unitaire de ladite région revêtue du substrat imperméable à l'eau allant de 2,05 × 10⁻⁸ mol/cm⁻² à 1,23 × 10⁻⁴ mol/cm², de préférence de 1,64 × 10⁻⁷ mol/cm² à 3,69 × 10⁻⁵ mol/cm² et plus préférablement de 4,92 × 10⁻⁷ mol/cm² à 8,20 × 10⁻⁶ mol/cm².

3. Article dépilatoire selon l'une ou l'autre revendication précédente, dans lequel la composition dépilatoire aqueuse comprend une base, de préférence une base tampon et de préférence dans un intervalle de concentration allant de 0,1 % à 10,0 %, plus préférablement de 0,5 % à 8 % et encore plus préférablement de 1 % à 5 % en poids de la composition dépilatoire aqueuse.

4. Article dépilatoire selon la revendication 3, dans lequel la base comprend un cation divalent, de préférence un cation métallique divalent, plus préférablement un cation calcium, un cation magnésium, un cation zinc ou leurs mélanges et encore plus préférablement un cation calcium.

5. Article dépilatoire selon l'une quelconque des revendications précédentes, dans lequel le sel thioglycolate comprend un cation divalent, de préférence un cation métallique divalent, plus préférablement un cation divalent zinc, calcium, magnésium, baryum ou strontium, ou leurs mélanges et encore plus préférablement un cation calcium.

6. Article dépilatoire selon l'une quelconque des revendications précédentes, dans lequel la concentration de l'acide conjugué du sel thioglycolate va de 0,5 % à 12,0 %, plus préférablement de 0,8 % à 8,0 % et encore plus préférablement de 1,0 % à 6,0 % en poids de la composition dépilatoire aqueuse.

7. Article dépilatoire selon l'une quelconque des revendications précédentes, dans lequel la composition dépilatoire aqueuse comprend des cations monovalents en une quantité par surface unitaire de la région revêtue du substrat imperméable à l'eau inférieure à 5,10 × 10⁻⁴ mol/cm², de préférence inférieure à 3 × 10⁻⁴ mol/cm⁻², plus préférablement de 1 × 10⁻⁹ mol/cm² à 1,5 × 10⁻⁴ mol/cm², encore plus préférablement de 2,50 × 10⁻⁸ mol/cm² à 6,65 × 10⁻⁵ mol/cm² et même plus préférablement encore de 6 × 10⁻⁷ mol/cm² à 4,5 × 10⁻⁵ mol/cm².

8. Article dépilatoire selon l'une quelconque des revendications précédentes, dans lequel la composition dépilatoire aqueuse comprend un silicate hydrosoluble choisi parmi : des silicates de lithium, des silicates de sodium (y compris, métasilicate disodique pentahydraté et métasilicate disodique nanohydraté), des sels de potassium, des silicates d'ammonium, des silicates d'imidazolium, des silicates synthétiques (argiles) ou leurs mélanges ; plus préférablement choisi parmi : des argiles synthétiques, des silicates de sodium, des silicates de potassium, ou leurs mélanges et encore plus préférablement dans lequel la composition dépilatoire aqueuse comprend un silicate de sodium ou des mélanges de silicates de sodium.

9. Article dépilatoire selon l'une quelconque des revendications précédentes, dans lequel la composition dépilatoire aqueuse comprend de l'eau en une quantité d'au moins 40 %, de préférence de 50 % à 98 %, plus préférablement de 60 % à 95 % et encore plus préférablement de 70 % à 90 % en poids de la composition dépilatoire aqueuse.

10. Article dépilatoire selon l'une quelconque des revendications précédentes, où l'article dépilatoire est un masque, une bande ou une pastille.

11. Article dépilatoire selon l'une quelconque des revendications précédentes, dans lequel ledit substrat imperméable à l'eau comprend un matériau imperméable à l'eau, de préférence une feuille plastique, plus préférablement une polyoléfine, encore plus préférablement un polyéthylène et même plus préférablement encore du polyéthylène à haute densité.

12. Article dépilatoire selon l'une quelconque des revendications précédentes, dans lequel une couche protectrice détachable (3) est fixée de manière amovible à la composition dépilatoire aqueuse.

13. Procédé d'élimination de poils de la peau, comprenant les étapes consistant à :
(a) appliquer un article dépilatoire selon l'une quelconque des revendications précédentes sur une surface de peau, de préférence la peau humaine,
(b) laisser ledit article dépilatoire en contact avec la peau pendant une période supérieure à 1 minute, de préférence 2 à 10 minutes, plus préférablement 2 à 8 minutes
(c) retirer ledit article dépilatoire de la surface de la peau, et
(d) de préférence frotter, racler, rincer ou essuyer la surface de la peau dans la zone sur laquelle l'article dépilatoire a été appliqué.

14. Trousse dépilatoire, comprenant :
(a) au moins un article dépilatoire selon l'une quelconque des revendications 1 à 12,
(b) facultativement, au moins un élément parmi une composition de soin de la peau de prétraitement, une composition de soin de la peau de post-traitement et/ou un outil pour faciliter le retrait des poils et/ou de la composition dépilatoire aqueuse après utilisation, et
(c) un conditionnement pour ladite trousse dépilatoire.
